# EUROPEAN PATENT SPECIFICATION

(11) **EP 0 254 315 B1**
(45) Date of publication and mention of the grant of the patent: **27.03.1996**
(21) Application number: 87110696.9
(22) Date of filing: 23.07.1987
(51) Int. Cl.: C12N 15/10, C12N 15/11, C12N 15/85, C12N 5/16, C12P 21/02

(54) **Mammalian cell derived autonomously replicating sequence DNA fragments having affinity to DNA binding proteins**
Autonom replizierende DNA Fragmente aus Säugerzellen mit Affinität zu DNA bindenden Proteinen
Fragments d'ADN a replication autonome, derivés de cellules de mammifères et ayant une affinité envers les proteines se fixant sur le ADN.

(30) Priority: 24.07.1986 JP 174036/86; 26.09.1986 JP 227455/86
(43) Date of publication of application: 27.01.1988
(73) Proprietor: DAIICHI PHARMACEUTICAL CO., LTD., Chuo-ku, Tokyo 103 (JP)
(72) Inventor: Ariga, Hiroyoshi, Tokyo (JP)
(74) Representative: Kinzebach, Werner, Dr.

(56) References cited:
- EP-A- 0 045 573
- EP-A- 0 240 373
- MOLECULAR & CELLULAR BIOLOGY, vol. 5, no. 3, March 1985, Washington, DC (US); H. ARIGA et al., pp. 563-568
- NUCLEIC ACIDS RESEARCH, vol. 12, no. 2, January 1984, Cambridge (GB); J.F. MONTIEL et al., pp. 1049-1068
- MOLECULAR & CELLULAR BIOLOGY, vol. 7, no. 1, January 1987, Washington, DC (US); H. ARIGA et al., pp. 1-6
- CHEMICAL ABSTRACTS, vol. 99, 1983, Columbus, OH (US); W. HSU et al., p. 134, no. 33863f
- EMBO JOURNAL, vol. 6, no. 8, August 1987, IRL Press Ltd., Oxford (GB); S.M.M. IGUCHI-ARIGA et al., pp. 2365-2371

## Description

### FIELD OF THE INVENTION

This invention relates to mammalian cell-derived autonomously replicating sequence DNA fragments having affinity to DNA-binding proteins, to plasmids containing those DNA fragments, to mammalian cells transfected with such a plasmid, to a method of producing peptides applying those transfected cells, and to a method of recovering mammalian cell-derived autonomously replicating sequence DNAs.

### BACKGROUND OF THE INVENTION

Known methods of producing peptides by utilizing genetic engineering techniques include, among others, causing microorganisms such as Escherichia coli and Bacillus subtilis to produce peptides, proteins and the like by utilizing plasmids and causing hosts to viruses to produce peptides, proteins and the like by utilizing viral DNA.

However, these known methods are not entirely satisfactory with respect to the stability of plasmids, the variety of usable cell species, and production efficiency.

The present inventor previously succeeded in isolating a mouse cell-derived DNA fragment containing an autonomously replicating sequence (ARS) and reported that this fragment is an EcoRI-BglII fragment of about 2,500 base pairs (Mol. Cell. Biol., 5, 563-568 (1985)).

J.M. Montiel et al provide in Nucl. Acid Res., Jan. 1984, 12(2), 1049-1068 a characterization of human chromosomal DNA sequences which replicate autonomously in Saccharomyces cerevisiae.

EP 0 240 373 which is a document relevant under Art 54(3) EPC discloses certain plasmids containing mammalian cell derived autonomously replicating sequence DNA fragments.

The present inventors carried out further investigations into the properties of this ARS fragment and found that the ARS has affinity for the c-myc protein which is the product of the c-myc gene and that the c-myc protein binds with c-myc gene itself and controls expression thereof. Furthermore the inventor found that not only myc protein but also various DNA-binding proteins have affinity for the ARS. This finding made it possible to separate and obtain the ARS from various mammalian cells using the myc protein and the like. The inventor further found that useful peptides, proteins or glycoproteins, among others, can be produced efficiently by utilizing such a mammalian cell-derived autonomously replicating sequence (ARS), and have now completed the present invention.

### SUMMARY OF THE INVENTION

The invention provides a method of recovering mammalian cell-derived autonomously replicating sequence DNAs which comprises binding a mammalian cell-derived DNA fragment to a DNA-binding protein, separating the binding product and isolating the DNA from the binding product; plasmids which contain a mammalian cell-derived autonomously replicating sequence DNA, a promoter and a gene for peptide production (inclusive of the translation initiation codon); mammalian cells transfected with these plasmids; and a method of producing peptides which comprises utilizing the mammalian cell-derived autonomously replicating sequence DNAs, the plasmids or the transformed cells.

### BRIEF DESCRIPTION OF THE DRAWINGS

In the accompanying drawings,
Figs. 1-4 each shows an outlined construction scheme for the respective plasmid, and
Figs. 5 to 7 each shows possible secondary structure of a certain DNA portion to be referred to later herein.
Fig. 8 shows comparison of the DNA sequences between three plasmids.

### DETAILED DESCRIPTION OF THE INVENTION

According to one embodiment of the present invention a mammalian cell-derived autonomously replicating sequence DNA fragment is provided having affinity for the human DNA-binding protein c-myc.

The autonomously replicating sequence is selected from the group consisting of the following DNA sequences: 
a)    GTATGATACAGATCGTGAGAATACGTAGCCTCGTCACCATTGAGCAGTAC
      GTTGTACTGGAAGAGACCATGCTCTGACACTGCACGACGTGACAGCATC

b)    AAGCTTGTTTTGGCCGTTTGTAGGGTTTGT
      TGGAATTTTTTTTCCGTCTGTGTACTTCG
      TCGAATTATTTCACGTTGCCATTACCGGTT
      CTCCATAGGGTGATGTTCATTAGCAGTTGG
      ATGATAGGTTATTCACATCTCTTATGGCGG
      TGAATAGTCACCTCTTGAACCACTTTTTCC
      TCCAGTAACTCCTCTTTCTTCGGACCTTCT
      GCAG

c)    CATTACCGGTTCTCCATAGGGTGATGTTCATTAGCAGTTGG
      ATGATAGGTTATTCACATCTCTTATGGCGGTAATAGTCACC
      TCTTGAACCACTTTT

d)    CATTACCGGTTCTCCATAGGGTGATGTTCATTAGCAGTTGG
      ATGATAGGTTATTCACATCTCTTATGGCGGTGAATAGT

e)    CATTACCGGTTCTCCATAGGGTGATG

f)    TATTCACATCTCTTATGGCGGTGAATAGT

g)    GTGAATAGTCACCTCTTGAACCACTTTT

h)    TATTCACATCTCTTATGGCGGTGAATAGTCACCTCTTGAACCACTTTT

i)    ATCGTGAGAATACGTAGCCTCGTCACCATTGAGCAGTACGTTGTACTG

k)    GAGAATACGTAGCCTCGTCACCATTGAGCAGTACGTTGTACT

      GGAAGAGACCATGCTC
 and functional equivalents thereto, having affinity for the DNA-binding protein as defined above.

Another embodiment relates to a method of recovering mammalian cell-derived autonomously replicating sequence DNA fragments which comprises binding a mammalian cell-derived DNA fragment to the DNA-binding protein, separating the DNA/DNA-binding protein complex and isolating the DNA from said DNA/ DNA-binding protein complex, wherein the DNA-binding protein is selected from the group consisting of myc proteins, c-myb protein, v-myb protein, c-fos protein, v-fos protein and p 53, and wherein the ARS DNA originates from a mammalian cell line producing said DNA-binding protein at a high level. Preferably, a nuclear extract of the cell line is employed as a DNA-binding protein source.

This method may be performed in a way, wherein
a) prior to the separation step the DNA/DNA-binding protein complex is further bound to an anti-DNA-binding protein antibody in order to obtain a DNA/DNA-binding protein/anti-DNA-binding protein antibody complex; and
b) the complex obtained from step a) is further bound to a substance capable of specifically binding to the antibody of the complex.

Preferably the substance as applied in step b) is selected from the group consisting of protein A and an antibody directed to the anti-DNA-binding protein antibody in the complex obtained from step a).

Another embodiment relates to a mammalian cell-derived autonomously replicating sequence DNA fragment, obtainable by a method as disclosed above.

According to the present invention plasmids are provided which contain a mammalian cell-derived autonomously replicating sequence DNA fragment as defined above, a promoter and a gene for peptide production inclusive of the translation initiation codon.

Another aspect of the present invention refers to a method of producing a peptide which comprises propagating in cells a plasmid containing a mammalian cell-derived autonomously replicating sequence DNA fragment as defined above, a promoter and a gene for peptide production inclusive of the translation initiation codon.

According to a further preferred embodiment of the present invention a plasmid is provided which contains a mammalian cell-derived autonomously replicating sequence DNA fragment as defined above,being obtainable by
a) treating the human c-myc gene with HindIII and PstI;
b) separating the upstream HindIII-PstI fragment; and
c) subcloning the fragment into pUC19 at the corresponding site.

Moreover,mammalian cell-derived autonomously replicating sequence DNA fragments are provided which are obtainable by
a) excising from the plasmid as obtained according to the above-mentioned method the HindIII-PstI fragment of about 200bp; and
b) separating the fragment by forming a complex which comprises the c-myc-protein.

The invention further relates to mammalian cells transfected with a plasmid as defined above.

The autonomous replicating sequence or ARS is the origin of replication in autonomous replication of eucaryotic chromosomes. The ARS can be obtained in the following manner: DNA is separated from mammalian cells and treated with an appropriate restriction enzyme or enzymes, preferably six-base restriction enzyme such as HindIII, EcoRI, BamHI, etc. The thus-obtained DNA fragments which are normally in the range of 1,000-10,000 base pairs in length are mixed with the DNA-binding protein to thereby form a DNA-binding protein complex.

In this specification and claims the DNA-binding protein is defined as the protein that binds with DNA, exists in the mammalian cell nucleus and controls replication or expression of the DNA in the cell, which may be called non-histone protein, and examples of the DNA-binding protein include myc proteins (such as c-myc protein (Science 225, 718-720 (1984)), v-myc protein (Nature 296, 262-264 (1982)), N-myc protein (Annu. Rev. Biochem., 52, 301-310 (1983)), c-myb protein, v-myb protein, c-fos protein, v-fos protein, p53 (each described in Annu. Rev. Biochem. 52 301-310 (1983)), SV40 T antigen (Cell 13 165 (1978)) and RB gene product (Science 235 1394-1399 (1987)). These proteins may be available in cell nuclear extract as described in the above references, for example, c-myc protein is available in HL-60 cell nuclear extract. For the separation procedure after formation of such DNA-protein complex, conventional means of separation or isolation of proteins, such as the procedure for antigen-antibody reactions or the like may be used.

For example, it is convenient that this complex is further bound to an antibody to the DNA-binding protein and the resultant complex is further bound to a substance capable of specifically binding to the antibody, for example, the second antibody or protein A, to produce a complex having molecular weight as large as forming precipitate. Precipitate of the complex can be collected with ease by a conventional manner such as filtration or centrifugation.

The antibody to the DNA-binding protein can be produced by a general manner such as by immunizing an animal with the protein but it is convenient to obtain from commercial sources such as Oncor Inc., U.S.A. and etc. For example, the third exon of N-myc gene (the specific moiety of N-myc) is expressed in E. coli to obtain N-myc protein then a mouse is immuninized with the protein by a general method. Anti-serum is collected from the mouse and the IgG fraction thereof is used as the anti-N-myc protein antibody. (see also J. Virol. 34 752-763 (1980)).

The second antibody can also be produced by a method known per se, and protein A are provided as a form of cell suspension of Staphylococcus aureus (P-7l55 (Sigma)) or a combined form with some solid (Protein A-Sepharose CL-4B (Pharmacia) or Protein A-Agarose FPA-l (Cosmo-Bio Ltd.).

Separation of the DNA from the complex (e.g., DNA=binding protein=antibody=protein A) can be carried out in a conventional manner, for example, by treatment with a reagent such as 2-mercaptoethanol or a proteolytic enzyme (Mol. Cell. Biol. 3, 1958-1966 (1983)) such as Proteinase K (Proteinase K P0390 (Sigma), Proteinase K No. 24568 (Merck)), however, 2-mercaptoethanol is preferable since the proteolytic enzyme may sometimes degradate DNA chain.

It is a very important fact discovered by the present inventor that the thus-isolated DNA fragment having specific affinity for the DNA-binding protein contains ARS DNA, and that the DNA-binding protein and ARS have very strong affinity for each other.

The ARS-containing DNA fragment can be utilized either in the form of ARS itself or of an ARS-containing DNA fragment having an appropriate length, as desired.

The size of possible ARS DNA is considered in about one hundred base pairs, however, it is also supposed that sometimes the ARS DNA is partially overlapped with an enhancer DNA. So, use can be made of a DNA having several hundreds base pairs as ARS-containing DNA fragment. The techniques of DNA treatment, screening and so on for producing useful substances, for example, peptides, by utilizing the ARS will be apparent and readily understandable to those with ordinary skill in the art when they use conventional or known techniques with reference to the examples given herein and so forth (T. Maniatis et al., Molecular Cloning, Cold Spring Harbor Laboratory (1982), hereinafter Mol. Clon.).

It is quite possible that the base sequence of the ARS might vary slightly depending on the species of animal used for providing cells from which the ARS is derived, depending on the cell species, or depending on the site which serves as the source of the ARS (when cells of the same strain are used). Therefore, it is not always necessary that an ARS to be used should be completely identical in base sequence to the ARS obtained in the above manner. On the contrary, it is possible to perform partial base substitution in or deletion from or, further, base addition to the ARS. Whether the resulting ARS is suited for efficient production of the desired product can be determined by conventional testing and study with reference to the description given herein.

Referring to the invention, as the isolable or usable ARS, an ARS derived from mammalian cells such as cells of a mouse, rat, guinea pig, cattle, horse, sheep, goat, rabbit, monkey, chimpanzee or human is exemplary. In particular, a mouse or human cell-derived ARS may be considered appropriate since advanced cell culture techniques are available for mouse and human cells.

Though any mammalian cells can be employed as an origin for the production of ARS, it is convenient to use such cells much producing the DNA-binding proteins as c-myc protein as described in Science 225, 718-720 (1984), v-myc protein described in Nature 296, 262-264 (1982), N-myc protein, c-myb protein, v-myb protein, c-fos protein, v-fos protein, p53 which are described in Annu. Rev. Biochem., 52, 301-310 (1983), SV40 T antigen described in Cell, 13, 165 (1978) and RB gene product described in Science, 235, 1394-1399 (1987).

Representative examples of such mammalian cells from which ARS is isolated include human HL-60 cell, human IMR-32 cell, human Raji cell, mouse FM3A cell and the like.

The ARS-containing plasmids according to the invention, which are suited for peptide production, can be constructed by utilizing conventional gene recombination techniques (cf. Mol. Clon.).

Introduction of the plasmids into cells, selection of the kind of cells to be used, cell proliferation and other necessary procedures can be carried out or made by utilizing those mammalian cell lines or strains and techniques that are generally known in the art. It will be also possible to utilize those techniques, cultured cells, media, and so forth which might be improved or developed in the future. (cf. "Manual of Cell Culture", edited by Munemura, Kodansha, Tokyo (1982), K. Habel and N. P. Salzman, Fundamental Technique in Virology, Academic Press, N.Y. (1969), Kruse and Patterson, Tissue Culture, Academic Press, N.Y. (1973) and Virology, 52 456-467 (1973)).

Several examples of the general procedures are given below for further illustration.

Examples of promoters which are usable are cell-derived promoters of various kinds, such as the thymidine kinase promoter and immunoglobulin promoter, and promoters from viruses infectious to mammalian cells, such as the SV40 T antigen promoter or SV40 early promoter, SV40 late promoter, Adenovirus promoters and the herpes virus thymidine kinase promoter. An ordinary ATG can be used as the translation initiation codon.

Examples of peptides to be produced include among others, those peptides, proteins, glycoproteins and the like that are usable as drugs, for example, insulin, growth hormone, interferons, tumor necrosis factor, interleukins, other lymphokines, and various enzymes.

Examples of genes for peptide production include among others, DNAs coding for respective amino acid sequences of the above-mentioned peptides, and sequences composed of the DNAs and a poly(A) signal for terminating translation as added thereto on the 3ʹ downstream side.

The plasmids according to the invention are produced by joining a DNA fragment having an appropriate number of base pairs and one or more sites cleavable with restriction enzymes in common use, a promoter, the translation initiation codon and a gene for peptide production in an appropriate, desired order, further adding an ARS and circularizing the construct.

The direction or location of the ARS need not be considered as particularly limited.

The plasmids thus obtained are introduced into various mammalian cells e.g., by transfection method using calcium phosphate, the microinjection method, the liposome method, the protoplast fusion method or the like, whereby transformed cells can be produced. Mouse NS-l and FM3A and human HL-60, U937, Daudi and Raji are examples of suitable cultured cells but not limited to those. The desired peptides can be produced by growing and multiplying these transformed cells.

A characteristic feature of the invention is that the cells into which the plasmids are to be introduced need not be from the same species as the species from which the ARS is derived. That is, when an ARS DNA is obtained from a mouse cell, the DNA can be used for the expression in another kind of mouse cells and in human cells or in other mammalian cells.

The transformed cells are grown and multiplied by conventional methods of cultivating the cells used, for example, in DMEM (Dulbecco modified Eagle's medium) containing an appropriate amount of fetal calf serum at 37°C in the presence of 5% carbon dioxide, or in the peritoneal cavity of an animal (cf. "Manual of Cell Culture", edited by Munemura, Kodansha, Tokyo (1982), Fundamental Technique in Virology, Tissue Culture).

The peptides produced, when they have been released into the medium, can be isolated by conventional fractionation techniques, for example, by centrifugation and/or gel filtration. When the peptides accumulate in cells, the peptides can be recovered by ordinary fractionation after disruption of the cells. Also when they are in the ascitic fluid, they can be isolated using conventional techniques.

The following examples are given to further illustrate the present invention but are by no means to be construed to be limitative thereof. Unless otherwise indicated, all parts, percents, ratios and the like are by weight.

### Example 1

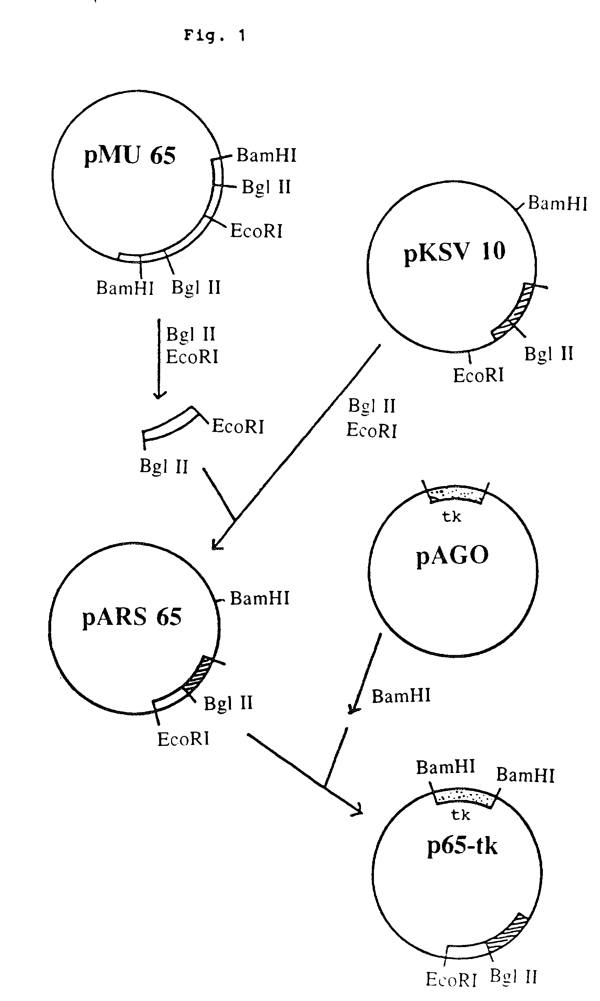

### (1) Construction of a Plasmid Containing Mouse ARS and the Thymidine Kinase Gene (cf. Fig. 1)

The plasmid pMU85 (Mol. Cell. Biol., 5 563-568 (1985)) was treated with EcoRI and BglII and a DNA fragment about 2,500 base pairs in length was isolated from this plasmid. This fragment was inserted into the plasmid pKSVl0 (commercially available from Pharmacia) in the EcoRI-BglII region thereof, whereby a plasmid, pARS65, was constructed.

The plasmid pAGO (Proc. Natl. Acad. Sci. USA, 76, 3755 (1979)) was treated with BamHI and a DNA fragment containing the herpes virus-derived thymidine kinase gene was isolated. This DNA fragment was inserted into the above-mentioned plasmid pARS65 at the BamHI site thereof. The thus-constructed plasmid p65-tk was allowed to multiply in E. coli (the transformant containing the plasmid E. coli K12 C600 ARS-1 having been deposited with the Fermentation Research Institute under the deposit number FERM BP-1443).

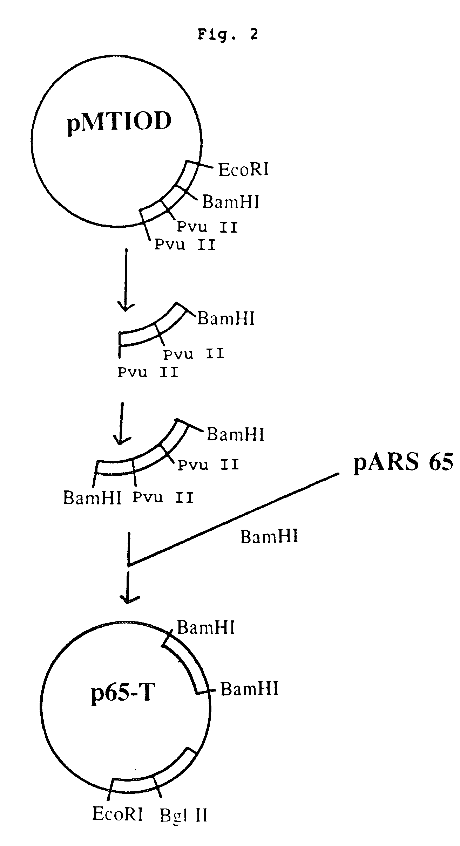

### (2) Construction of a Plasmid Containing Mouse ARS and the SV40 T Antigen Gene (cf. Fig. 2)

The plasmid pMTIOD (J. Virology, 48, 48l-49l (1981)) was treated with BamHI and PvuII and the SV40 T antigen gene with the early promoter therefor was isolated. The BamHI linker (Takara Shuzo, Japan) was joined to the PvuII site of this gene. The fragment thus obtained was inserted into the plasmid pARS65 at the BamHI site thereof to obtain a plasmid, p65-T.

### (3) Expression of Protein (thymidine kinase)

The plasmid p65-tk obtained in process (1) above was used to transfect FM3A^{tk-} cells by the liposome fusion method. The plasmid p65-tk was encapsulated in large unilamellar vesicles (LUVs) prepared by the phosphatidylserine calcium induced fusion case. In performing the transformation, 20 mM Tris buffer (pH 7.5) containing 50 mM EDTA was used (cf. Science 2l5, 166 (1982) and Proc. Natl. Acad. Sci. USA, 75, 4194 (1978).

After transformation, the cells were grown in DMEM containing l0% fetal calf serum at 37°C in the presence of 5% carbon dioxide. After 2 days, the medium was replaced with HAT medium.

Transformed cells, namely FM3A^{tk+} cells, were observed in approximately a week. After 2 weeks of cultivation, each colony was isolated and transferred to HAT medium. For each colony, cultivation in HAT medium was continued until the number of cells amounted to about l0⁷ per colony. The number of doublings during this cultivation period amounted to about 60.

One thousand cells were subjected to transformation by the above method, whereupon about 300-400 transformant cells were obtained. It was thus confirmed that p65-tk had propagated in FM3A^{tk-} cells and the thymidine kinase gene had been expressed.

### (4) Checking for Copy Number

The above-mentioned l0⁷ cells were extracted and obtained low molecular weight DNA fraction by the Hirt method (J. Mol. Biol., 26, 365-369 (1967)). To confirm plasmid replication in the transformants, the low-molecular-weight DNA fraction was treated with the restriction enzyme DpnI.

While p65-tk introduced into FM3A^{tk-} cells contains a methylated adenine moiety, the replication product yielded in these cells did not contain any methylated adenine group. Since DpnI cleaves methylated DNA selectively, it cleaves exclusively the plasmid introduced into the cells to give a plurality of DNA fragments, with the plasmid copies replicated in the cells remaining uncleaved. Therefore, the plasmid copies replicated in the cells can be easily distinguished by electrophoresis.

After DpnI digestion, DNAs were separated by agarose gel electrophoresis (Mol. Clon.) and subjected to Southern blot testing (J. Mol. Biol., 98, 503-517 (1975)). Thus, hybridization was carried out using ³P-labeled p65-tk as a probe, followed by X-ray autoradiography. Detection of bands hybridized to the probe confirmed that p65-tk had been replicated as an extrachromosomal DNA in all of the 20 clones investigated.

The copy number per cell estimated from the band intensity was l00-200. This plasmid was replicable in a stable manner also when the above cells were grown in DMEM containing l0% fetal calf serum, which was used in lieu of HAT medium. No modification was noted in the DNA level. It was also confirmed that this plasmid remained in the cells in a stable manner even after the lapse of the time for 150 doublings.

### (5) Replication of Plasmid pARS-65 in Different Cell Lines

pARS-65 was transfected into mouse NS-1 cells, human HL-60 cells, and human U937 cells, respectively, using the method described above. The cells were then grown in RPMI l640 medium containing l0% fetal calf serum at 37°C in the presence of 5% carbon dioxide. After 40 hours, the extrachromosomal low-molecular-weight DNA was analyzed by the method mentioned above in (4). As a result, about 500 copies of pARS-65 were confirmed to have been replicated in each NS-1 cell, about 10,000 copies were confirmed to have been replicated in each HL-60 cell, and about 100 copies were confirmed to have been replicated in each U937 cell.

It was thus confirmed that pARS-65 is replicable also in cells of other species than mouse. At the same time, the results suggest that p65-tk might have such replicability.

### Example 2

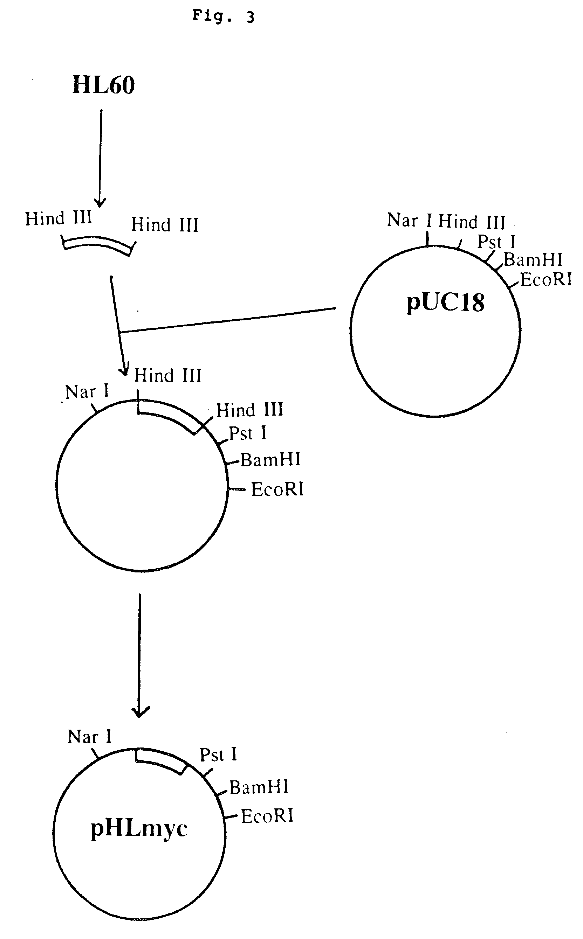

### 1) Isolation of Human Cell-Derived ARS (cf. Fig. 3)

Human HL-60 cell DNA was extracted using the SDS-proteinase K method (Mol. Clon.) and treated with HindIII. A DNA fragment having affinity for the myc protein was obtained from the DNA fragment mixture thus obtained, using a conventional method (Mol. Cell. Biol., 3, 1958-1966 (1983)) as follows:

The above fragment mixture was mixed with an HL-60 nuclear extract (rich in myc protein), and the reaction was allowed to proceed at 0°C for 30 minutes, yielding a DNA-myc protein complex.

Then, an anti-myc protein antibody (α-myc, commercially available from Oncor Inc. U.S.A.) was added, whereby a DNA-myc protein-α myc complex was formed. Furthermore, an aqueous solution of Staphylococcus aureus, which contained protein A capable of specifically binding to α myc, was added to form a DNA-myc protein-α myc-protein A complex.

This complex, which occurred as a precipitate, was collected by centrifuging, washed with Tris buffer (pH 7.5) containing 0.l% SDS and 0.l M sodium chloride, and then the DNA was released by adding a 7 mM aqueous mercaptoethanol solution containing l% SDS and allowing the reaction to proceed at 30°C for 30 minutes. The reaction mixture was centrifuged at l5,000 × g for 5 minutes. The supernatant thus separated was extracted with phenol to remove proteins. The desired DNA fragment thus obtained was inserted into pUCl9 (commercially available from Pharmacia) at the HindIII site thereof. The number of base pairs in the above DNA fragment was estimated at about 200 by agarose gel electrophoresis.

The plasmid obtained was multiplied in E. coli (the transformant with this plasmid E. coli K12 C600 ARS-2 having been deposited with the Fermentation Research Institute under the deposit number FERM BP-1444).

The above-described HL-60 nuclear extract was prepared in the following manner. Thus, l liter of HL-60 cell culture fluid (5 × l0⁵ cells/ml) was centrifuged and the cells harvested were washed with phosphate-buffered physiological saline. The cells were further washed with a hypotonic aqueous solution (20 mM HEPES, pH 7.5, 5 mM potassium chloride, 0.05 mM magnesium chloride, 0.5 mM dithiothreitol, 0.2 mM sucrose) and then suspended in a hypotonic aqueous solution (the former solution without any sucrose), and the suspension was allowed to stand for l0 minutes. After homogenization by 40 strokes in a Dounce homogenizer, the homogenate was centrifuged at 3,000 × g for l0 minutes to obtain a precipitate.

The precipitate comprising cell nuclei was dissolved in 2.5 ml of an aqueous solution (5 mM HEPES, pH 7.5, l0% sucrose) and stored temporarily in liquid nitrogen. After thawing this gradually at 0°C, 5 M aqueous sodium chloride was added to a final concentration of 0.1 M and, after 5 minutes of treatment at 0°C, the mixture was centrifuged at 15,000 × g for 20 minutes to obtain the nuclear extract as a supernatant.

The above-mentioned plasmid as multiplied in E. coli K12 C600 was isolated (Mol. Clon.). Treatment with HindIII failed to cleave this plasmid. Hence it was considered that bacterial rearrangement of the plasmid had occurred. This plasmid was designated pHLmyc.

The plasmid pUCl9 has other restriction enzyme cleavage sites (e.g. BamHI and NarI sites) than the HindIII site. Therefore, the above plasmid was treated with BamHI and NarI, and the smaller, in molecular weight of the two DNA fragments thus formed was isolated.

A ³P-labeled probe was prepared using this smaller fragment as the template and used for Southern hybridization (J. Mol. Biol., 98, 503-517 (1975)) to the HL-60 cell DNA. This probe hybridized to the HL-60 cell DNA and it was thus confirmed that the fragment having an NarI site at one end and a BamHI site at the other (NarI-BamHI fragment) contained an HL-60 cell-derived DNA.

The length of the NarI-BamHI fragment as estimated by agarose gel electrophoresis was about 200-300 based pairs.

In pUCl9, the HindIII site is l3l base pairs apart from the NarI site and 35 base pairs apart from the BamHI site, and a PstI site is present 25 base pairs apart from the BamHI site. In the plasmid pHLmyc, the PstI site was retained. Therefore, the number of base pairs in the HL-60 cell-derived DNA contained in the plasmid pHLmyc was estimated to be about 120.

This base sequence was determined and 99 bases were identified in the HindIII site within the polylinker region. The sequence thereof is shown below:

1
GTATGATACAGATCGTGAGAATACGTAGCCTCGTCACCATTGAGCAGTA
CGTTGTACTGGAAGAGACCATGCTCTGACACTGCACGACGTGACAGCATC
99

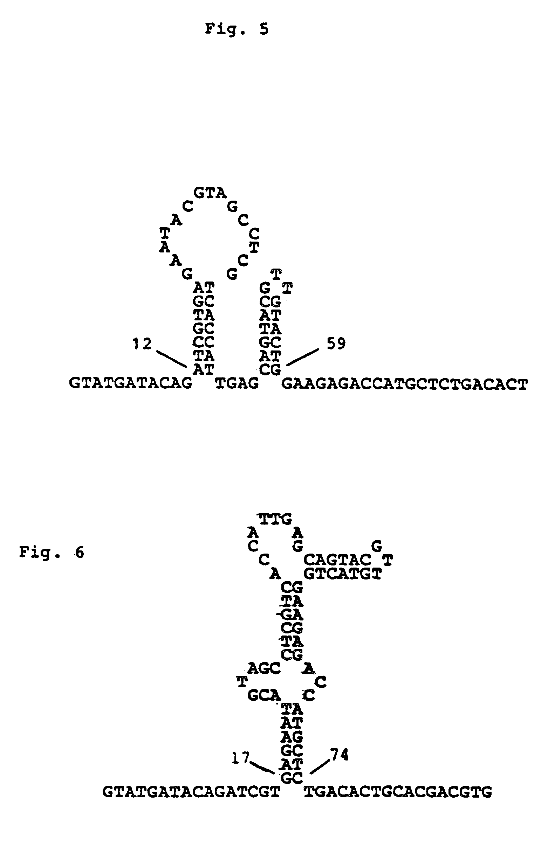

Examination of this sequence for inverted repeats suggested the possibility that it might have hairpin structures as shown in Fig. 5 and Fig. 6. On that basis, it was presumed, of the above 99 bases, bases 12 to 59 or bases 17 to 74 constituted the most important portion of the autonomously replicating sequence (ARS).

### (2) Confirmation of Plasmid pHLmyc Containing ARS

HL-60 cells were transfected with pHLmyc by the liposome method, and the copy number was estimated by the method described above and found to be 10,000 per cell.

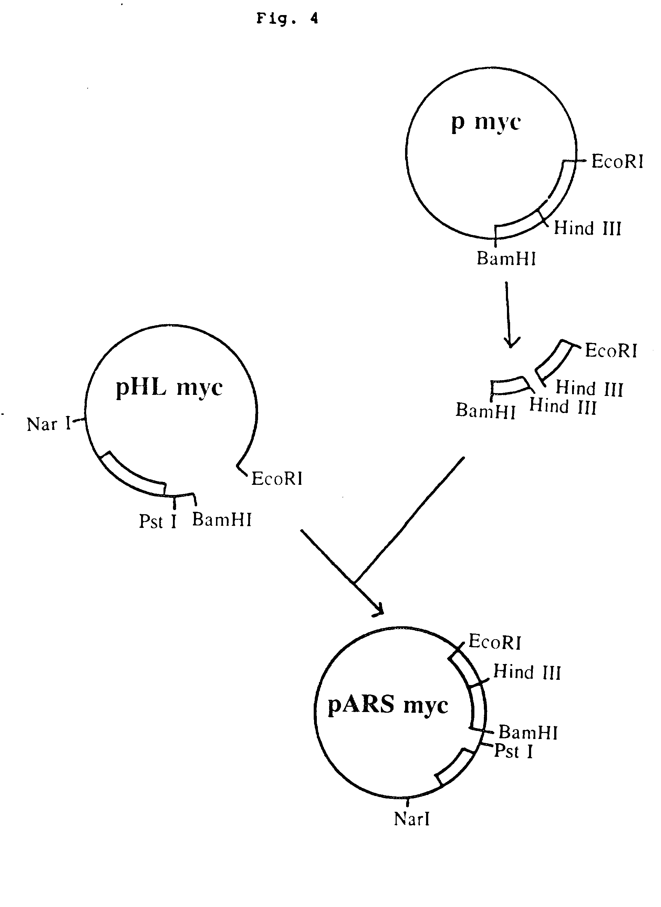

### (3) Construction of Plasmid Containing Human ARS and c-myc and Expression Thereof (cf. Fig. 4)

The plasmid pmyc (commercially available from Oncor Inc. U.S.A.; containing the myc structural gene and the Rous sarcoma virus-derived long terminal repeat promoter (LTR)) was treated with BamHI, HindIII and EcoRI, and the myc structural gene and LTR were isolated and inserted into pHLmyc in the EcoRI-BamHI region thereof. The thus-constructed plasmid pARSmyc was transfected into human U937 cells by the liposome method and the cells were grown in RPMI l640 medium containing l0% fetal calf serum at 37°C in the presence of 5% carbon dioxide.

After 3 days of incubation, the quantity of mRNA corresponding to the myc gene as occurring in the cells was determined by the Northern hybridization method (Mol. Clon.) and found to be about l00 times greater as compared with the quantity of the mRNA in pARS-free U937 cells. Therefore, it was concluded that mRNA transcription from the myc gene in the plasmid pARSmyc had taken place.

### Example 3

(1) Sub-Cloning of Human c-myc Gene
   Human c-myc gene (Mol. Cell Biol., 5, 414-418 (1985)) was digested with HindIII and KpnI and the upstream HindIII-KpnI region (about 1,200 bp) was separated. Similarly, the upstream HindIII-PstI fragment was obtained by treating the gene with HindIII and PstI. These fragments were subcloned into pUC18 and pUC19 at the corresponding site, respectively, and the resultant plasmids of the clone were designated pmyc (H-K) and pmyc (H-P), respectively.
(2) Binding of the HindIII-KpnI Fragment and HindIII-PstI Fragment with c-myc Protein
   Gel-shift assay method has been recently employed to examine binding of DNA with a protein (Nucleic Acid Res., 9, 3047-3060 (1981)). In the method, an isotope-labelled DNA is treated with a protein to bind and eluted on polyacrylamide gel, so the DNA-protein complex is late to move on the gel.
   The nuclear extract of HL-60 cell which was much producing c-myc protein was used as the c-myc protein source. Each HindIII-KpnI fragment and HindIII-PstI fragment described above was mixed with the nuclear extract and warmed at 30°C for 15 minutes. The resulting mixture was eluted on polyacrylamide gel. On the other hand, the nuclear extract was pre-treated with anti-myc protein antibody (Oncor Inc. U.S.A), which was then treated in the same manner as above with the above fragments, respectively.
   The each fragment was bound with protein, however, pretreated protein with antibody were not bound with the fragment. From the fact it was concluded that there is a binding site with the c-myc protein in the HindIII-PstI fragment (about 200 bp).
(3) Plasmid pmyc (H-K) was digested with HindIII and KpnI, whereas pmyc (H-P) was digested with HindIII and PstI to produce DNA fragments. Each fragment was treated similarly to Example 2 with c-myc protein, antibody and protein A in turn and the complexes were treated with 2-mercaptoethanol to cause protein degradation and to remove resulted proteins.

Thus, the HindIII-KpnI fragment and the HindIII-PstI fragment were obtained and DNA sequence of the HindIII-PstI fragment was determined by the dideoxy chain termination method, which is shown below.

1                            30
AAGCTTGTTTTGGCCGTTTGTAGGGTTTGT
TGGAATTTTTTTTCCGTCTGTGTACTTCG
TCGAATTATTTCACGTTGCCATTACCGGTT
CTCCATAGGGTGATGTTCATTAGCAGTTGG
ATGATAGGTTATTCACATCTCTTATGGCGG
TGAATAGTCACCTCTTGAACCACTTTTTCC
TCCAGTAACTCCTCTTTCTTCGGACCTTCT
GCAG

A possible secondary structue of the sequence was estimated by computor-assisted sequence analysis and is shown in Figure 7. Figure 8 shows comparison of the sequence between the fragment of pHLmyc and pmyc (H-P) and between the fragment of pARS65 and pmyc (H-P). Stars indicates matched sequences and sequence with bar is supposed to make stem of hairpin loop. The DNA sequence to make the stem, especially the nine base pairs of GTGAATAGT is considered most important.

### Example 4

Similarly to the procedure in Example 2, several plasmids were obtained from various cell nuclear DNA, proteins and antibodies. The name of plasmid and the raw materials were shown in the following table.

| Plasmid | DNA/Enzyme Used | Nuclear Extract of (Protein) | Antibody |
|---|---|---|---|
| pIMR-N | IMR 32 DNA/HindIII | IMR 32 cell (N-myc protein) | anti-N-myc protein |
| pRJ-53 | Raji DNA/HindIII | Raji cell (p53) | anti p53 |
| pHLmyc (Ex. 2) | HL-60 DNA/HindIII | HL-60 cell (c-myc protein) | anti-c-myc protein |

### Identification of ARS

Plasmids pmyc (H-K), pmyc (H-P), pIMR-N and pRJ-53 were introduced into cell in a similar manner to Example l (3) and the copy number was investigated. The results are shown below.

| Plasmid | Cell | Copy Number |
|---|---|---|
| pmyc (H-K) | HL60 | 1000-5000 |
| pmyc (H-P) | HL60 | 1000-5000 |
| pIMR-N | IMR-32 | 1000-5000 |
| pRJ-53 | Raji | 1000-5000 |

As described in detail hereinabove, the plasmids according to the invention can be replicated with good efficiency in various mammalian cells due to the function of the ARS; the plasmid copy number per cell is high and the gene product production efficiency is high. Therefore, the present invention also provides a peptide production which is excellent from the genetic engineering viewpoint.

While the invention has been described in detail and with reference to specific embodiments thereof, it will be apparent to one skilled in the art that various changes and modifications can be made therein without departing from the spirit and scope thereof.

## Claims (Claims for the following Contracting State(s): BE, CH, DE, FR, GB, GR, IT, LI, NL, SE)

1. A method of recovering a mammalian cell-derived autonomously replicating sequence (ARS) DNA fragment which comprises binding a mammalian cell-derived DNA fragment to a DNA-binding protein, separating the DNA/DNA-binding protein complex and isolating the DNA from said DNA/DNA-binding protein complex,
wherein the DNA-binding protein is selected from the group consisting of myc proteins, c-myb protein, v-myb protein, c-fos protein, v-fos protein and p 53, and wherein the ARS DNA originates from a mammalian cell line producing said DNA-binding protein at a high level.

2. The method of claim 1, wherein
a) prior to the separation step the DNA/DNA-binding protein complex is further bound to an anti-DNA-binding protein antibody in order to obtain a DNA/DNA-binding protein/anti-DNA-binding protein antibody complex; and
b) the complex obtained from step a) is further bound to a substance capable of specifically binding to the antibody of the complex.

3. The method of claim 2, wherein the substance as applied in step b) is selected from the group consisting of protein A and an antibody directed to the anti-DNA-binding protein antibody in the complex obtained from step a).

4. The method of one of the claims 1 to 3, wherein a nuclear extract of a cell line is employed as a DNA-binding protein source.

5. A human cell-derived autonomously replicating sequence DNA fragment having affinity for the human DNA-binding protein c-myc, selected from the group consisting of the following sequences: 
a)	GTATGATACAGATCGTGAGAATACGTAGCCTCGTCACCATTGAGCAGTAC
	GTTGTACTGGAAGAGACCATGCTCTGACACTGCACGACGTGACAGCATC

b)	AAGCTTGTTTTGGCCGTTTGTAGGGTTTGT
	TGGAATTTTTTTTCCGTCTGTGTACTTCG
	TCGAATTATTTCACGTTGCCATTACCGGTT
	CTCCATAGGGTGATGTTCATTAGCAGTTGG
	ATGATAGGTTATTCACATCTCTTATGGCGG
	TGAATAGTCACCTCTTGAACCACTTTTTCC
	TCCAGTAACTCCTCTTTCTTCGGACCTTCT
	GCAG

c)	CATTACCGGTTCTCCATAGGGTGATGTTCATTAGCAGTTGG
	ATGATAGGTTATTCACATCTCTTATGGCGGTAATAGTCACC
	TCTTGAACCACTTTT

d)	CATTACCGGTTCTCCATAGGGTGATGTTCATTAGCAGTTGG
	ATGATAGGTTATTCACATCTCTTATGGCGGTGAATAGT

e)	CATTACCGGTTCTCCATAGGGTGATG

f)	TATTCACATCTCTTATGGCGGTGAATAGT

g)	GTGAATAGTCACCTCTTGAACCACTTTT

h)	TATTCACATCTCTTATGGCGGTGAATAGTCACCTCTTGAACCACTTTT

i)	ATCGTGAGAATACGTAGCCTCGTCACCATTGAGCAGTACGTTGTACTG

k)	GAGAATACGTAGCCTCGTCACCATTGAGCAGTACGTTGTACT

	GGAAGAGACCATGCTC
 and functional equivalents thereto.

6. A plasmid which contains a mammalian cell-derived autonomously replicating sequence DNA fragment according to claim 5, a promoter and a gene for peptide production inclusive of the translation initiation codon.

7. A method of producing a peptide which comprises propagating in cells a plasmid containing a mammalian cell-derived autonomously replicating sequence DNA fragment according to claim 5, a promoter and a gene for peptide production inclusive of the translation initiation codon.

8. A mammalian cell transfected with a plasmid containing a mammalian cell-derived autonomously replicating sequence DNA fragment according to claim 5, a promoter and a gene for peptide production inclusive of the translation initiation codon.

9. A plasmid which contains a mammalian cell-derived autonomously replicating sequence DNA fragment, obtainable by
a) treating the human c-myc gene with HindIII and PstI;
b) separating the upstream HindIII-PstI fragment; and
c) subcloning the fragment into pUC19 at the corresponding site.

10. A mammalian cell-derived autonomously replicating sequence DNA fragment, obtainable by,
a) excising from the plasmid obtainable according to claim 9 the HindIII-PstI fragment of about 200 bp; and
b) separating the fragment by forming a complex which comprises the c-myc protein.

11. The ARS DNA fragment of claim 5 or 10, characterized by the sequence GTGAATAGT.

12. A method of producing a peptide which comprises propagating in cells a plasmid according to claim 9.

13. A mammalian cell transfected with a plasmid according to claim 9.

## Claims (Claims for the following Contracting State(s): AT, ES)

1. A method of recovering a mammalian cell-derived autonomously replicating sequence (ARS) DNA fragment which comprises binding a mammalian cell-derived DNA fragment to a DNA-binding protein, separating the DNA/DNA-binding protein complex and isolating the DNA from said DNA/DNA-binding protein complex,
wherein the DNA-binding protein is selected from the group consisting of myc proteins, c-myb protein, v-myb protein, c-fos protein, v-fos protein and p 53, and wherein the ARS DNA originates from a mammalian cell line producing said DNA-binding protein at a high level.

2. The method of claim 1, wherein
a) prior to the separation step the DNA/DNA-binding protein complex is further bound to an anti-DNA-binding protein antibody in order to obtain a DNA/DNA-binding protein/anti-DNA-binding protein antibody complex; and
b) the complex obtained from step a) is further bound to a substance capable of specifically binding to the antibody of the complex.

3. The method of claim 2, wherein the substance as applied in step b) is selected from the group consisting of protein A and an antibody directed to the anti-DNA-binding protein antibody in the complex obtained from step a).

4. The method of one of claims 1 to 3, wherein a nuclear extract of a cell line is employed as a DNA-binding protein source.

5. The method of one of claims 1 to 4, wherein a human cell-derived autonomously replicating sequence DNA fragment having affinity for the human DNA-binding protein c-myc is obtained, selected from the group consisting of the following sequences: 
a)	GTATGATACAGATCGTGAGAATACGTAGCCTCGTCACCATTGAGCAGTAC
	GTTGTACTGGAAGAGACCATGCTCTGACACTGCACGACGTGACAGCATC

b)	AAGCTTGTTTTGGCCGTTTGTAGGGTTTGT
	TGGAATTTTTTTTCCGTCTGTGTACTTCG
	TCGAATTATTTCACGTTGCCATTACCGGTT
	CTCCATAGGGTGATGTTCATTAGCAGTTGG
	ATGATAGGTTATTCACATCTCTTATGGCGG
	TGAATAGTCACCTCTTGAACCACTTTTTCC
	TCCAGTAACTCCTCTTTCTTCGGACCTTCT
	GCAG

c)	CATTACCGGTTCTCCATAGGGTGATGTTCATTAGCAGTTGG
	ATGATAGGTTATTCACATCTCTTATGGCGGTAATAGTCACC
	TCTTGAACCACTTTT

d)	CATTACCGGTTCTCCATAGGGTGATGTTCATTAGCAGTTGG
	ATGATAGGTTATTCACATCTCTTATGGCGGTGAATAGT

e)	CATTACCGGTTCTCCATAGGGTGATG

f)	TATTCACATCTCTTATGGCGGTGAATAGT

g)	GTGAATAGTCACCTCTTGAACCACTTTT

h)	TATTCACATCTCTTATGGCGGTGAATAGTCACCTCTTGAACCACTTTT

i)	ATCGTGAGAATACGTAGCCTCGTCACCATTGAGCAGTACGTTGTACTG

k)	GAGAATACGTAGCCTCGTCACCATTGAGCAGTACGTTGTACT

	GGAAGAGACCATGCTC
 and functional equivalents thereto.

6. A method of preparing a plasmid, which method comprises providing a plasmid which contains a mammalian cell-derived autonomously replicating sequence DNA fragment according to claim 5, a promoter and a gene for peptide production inclusive of the translation initiation codon.

7. A method of producing a peptide which comprises propagating in cells a plasmid containing a mammalian cell-derived autonomously replicating sequence DNA fragment according to claim 5, a promoter and a gene for peptide production inclusive of the translation initiation codon.

8. A method of preparing a transfected mammalian cell, which method comprises transfection of a mammalian cell with a plasmid containing a mammalian cell-derived autonomously replicating sequence DNA fragment according to claim 5, a promoter and a gene for peptide production inclusive of the translation initiation codon.

9. A method of preparing a plasmid which contains a mammalian cell-derived autonomously replicating sequence DNA fragment, which method comprises the steps of
a) treating the human c-myc gene with HindIII and PstI;
b) separating the upstream HindIII-PstI fragment; and
c) subcloning the fragment into pUC19 at the corresponding site.

10. A method of preparing a mammalian cell-derived autonomously replicating sequence DNA fragment, which method comprises the steps of
a) excising from the plasmid obtainable according to claim 9 the HindIII-PstI fragment of about 200 bp; and
b) separating the fragment by forming a complex which comprises the c-myc protein.

11. The method of claim 5 or 10, wherein an ARS DNA fragment, characterized by the sequence GTGAATAGT, is obtained.

12. A method of producing a peptide which comprises propagating in cells a plasmid according to claim 9.

13. A method of preparing a transfected mammalian cell which method comprises transfecting a mammlian cell with a plasmid according to claim 9.

## Patentansprüche (Patentansprüche für folgende(n) Vertragsstaat(en): BE, CH, DE, FR, GB, GR, IT, LI, NL, SE)

1. Verfahren zur Gewinnung eines von Säugerzellen abgeleiteten Autonom Replizierende Sequenz (ARS)-DNA-Fragmentes, wobei man ein von Säugerzellen abgeleitetes DNA-Fragment an ein DNA-Bindungsprotein bindet, den DNA/DNA-Bindungsprotein-Komplex abtrennt und die DNA aus dem DNA/DNA-Bindungsprotein-Komplex isoliert, wobei das DNA-Bindungsprotein ausgewählt ist unter myc-Proteinen, c-myb-Protein, v-myb-Protein, c-fos-Protein, v-fos-Protein und p 53 und wobei die ARS-DNA aus einer Säugerzellinie stammt, welche das erwähnte DNA-Bindungsprotein in erhöhter Menge produziert.

2. Verfahren nach Anspruch 1, wobei
a) der DNA/DNA-Bindungsprotein-Komplex vor dem Trennungsschritt an einen Anti-DNA-Bindungsprotein-Antikörper gebunden wird, um einen DNA/DNA-Bindungsprotein/Anti-DNA-Bindungsprotein-Antikörper-Komplex zu erhalten; und
b) der in Stufe a) erhaltene Komplex an eine Substanz gebunden wird, die in der Lage ist, spezifisch an den Antikörper des Komplexes zu binden.

3. Verfahren nach Anspruch 2, wobei die gemäß Stufe b) eingesetzte Substanz ausgewählt ist unter Protein A und einem Antikörper, der gegen den Anti-DNA-Bindungsprotein-Antikörper in dem in Stufe a) erhaltenen Komplex gerichtet ist.

4. Verfahren nach einem der Ansprüche 1 bis 3, wobei ein Kernextrakt einer Zellinie als Quelle für das DNA-Bindungsprotein verwendet wird.

5. Von Humanzellen abgeleitetes Autonom Replizierende Sequenz-DNA-Fragment mit Affinität gegenüber dem menschlichen DNA-Bindungsprotein c-myc, welches unter folgenden Sequenzen: 
a) GTATGATACAGATCGTGAGAATACGTAGCCTCGTCACCATTGAGCAGTAC
GTTGTACTGGAAGAGACCATGCTCTGACACTGCACGACGTGACAGCATC

b) AAGCTTGTTTTGGCCGTTTGTAGGGTTTGT
TGGAATTTTTTTTCCGTCTGTGTACTTCG
TCGAATTATTTCACGTTGCCATTACCGGTT
CTCCATAGGGTGATGTTCATTAGCAGTTGG
ATGATAGGTTATTCACATCTCTTATGGCGG
TGAATAGTCACCTCTTGAACCACTTTTTCC
TCCAGTAACTCCTCTTTCTTCGGACCTTCT
GCAG

c) CATTACCGGTTCTCCATAGGGTGATGTTCATTAGCAGTTGG
ATGATAGGTTATTCACATCTCTTATGGCGGTAATAGTCACC
TCTTGAACCACTTTT

d) CATTACCGGTTCTCCATAGGGTGATGTTCATTAGCAGTTGG
ATGATAGGTTATTCACATCTCTTATGGCGGTGAATAGT

e) CATTACCGGTTCTCCATAGGGTGATG

f) TATTCACATCTCTTATGGCGGTGAATAGT

g) GTGAATAGTCACCTCTTGAACCACTTTT

h) TATTCACATCTCTTATGGCGGTGAATAGTCACCTCTTGAACCACTTTT

i) ATCGTGAGAATACGTAGCCTCGTCACCATTGAGCAGTACGTTGTACTG

k) GAGAATACGTAGCCTCGTCACCATTGAGCAGTACGTTGTACT

GGAAGAGACCATGCTC
 und deren funktionellen Äquivalenten ausgewählt ist.

6. Plasmid, welches ein von Säugerzellen abgeleitetes Autonom Replizierende Sequenz-DNA-Fragment gemäß Anspruch 5, einen Promoter und ein Gen zur Peptidproduktion, einschließlich des Translationsinitiationskodons, enthält.

7. Verfahren zur Herstellung eines Peptids, wobei man in Zellen ein Plasmid propagiert, das ein von Säugerzellen abgeleitetes Autonom Replizierende Sequenz-DNA-Fragment gemäß Anspruch 5, einen Promoter und ein Gen zur Peptidproduktion, einschließlich des Translationsinitiationskodons, enthält.

8. Säugerzelle, die mit einem Plasmid transfiziert ist, welches ein von Säugerzellen abgeleitetes Autonom Replizierende Sequenz-DNA-Fragment gemäß Anspruch 5, einen Promoter und ein Gen zur Peptidproduktion, einschließlich des Translationsinitiationskodons, enthält.

9. Plasmid, welches ein von Säugerzellen abgeleitetes Autonom Replizierende Sequenz-DNA-Fragment enthält und welches erhältlich ist durch:
a) Behandlung des menschlichen c-myc-Gens mit HindIII und PstI;
b) Abtrennung des stromaufwärts gelegenen HindIII-PstI-Fragmentes; und
c) Subklonierung des Fragmentes in pUC19 an der entsprechenden Stelle.

10. Von Säugerzellen abgeleitetes Autonom Replizierende Sequenz-DNA-Fragment, erhältlich durch:
a) Ausschneiden des HindIII-PstI-Fragmentes mit einer Größe von etwa 200 bp aus dem nach Anspruch 9 erhältlichen Plasmid; und
b) Abtrennung des Fragmentes durch Bildung eines Komplexes, der das c-myc-Protein umfaßt.

11. ARS-DNA-Fragment gemäß Anspruch 5 oder 10, gekennzeichnet durch die Sequenz GTGAATAGT.

12. Verfahren zur Herstellung eines Peptids, welches die Propagierung eines Plasmids gemäß Anspruch 9 in Zellen umfaßt.

13. Säugerzelle, die mit einem Plasmid gemäß Anspruch 9 transfiziert ist.

## Patentansprüche (Patentansprüche für folgende(n) Vertragsstaat(en): AT, ES)

1. Verfahren zur Gewinnung eines von Säugerzellen abgeleiteten Autonom Replizierende Sequenz (ARS)-DNA-Fragmentes, wobei man ein von Säugerzellen abgeleitetes DNA-Fragment an ein DNA-Bindungsprotein bindet, den DNA/DNA-Bindungsprotein-Komplex abtrennt und die DNA aus dem DNA/DNA-Bindungsprotein-Komplex isoliert, wobei das DNA-Bindungsprotein ausgewählt ist unter myc-Proteinen, c-myb-Protein, v-myb-Protein, c-fos-Protein, v-fos-Protein und p 53 und wobei die ARS-DNA aus einer Säugerzellinie stammt, welche das erwähnte DNA-Bindungsprotein in erhöhter Menge produziert.

2. Verfahren nach Anspruch 1, wobei
a) der DNA/DNA-Bindungsprotein-Komplex vor dem Trennungsschritt an einen Anti-DNA-Bindungsprotein-Antikörper gebunden wird, um einen DNA/DNA-Bindungsprotein/Anti-DNA-Bindungsprotein-Antikörper-Komplex zu erhalten; und
b) der in Stufe a) erhaltene Komplex an eine Substanz gebunden wird, die in der Lage ist, spezifisch an den Antikörper des Komplexes zu binden.

3. Verfahren nach Anspruch 2, wobei die gemäß Stufe b) eingesetzte Substanz ausgewählt ist unter Protein A und einem Antikörper, der gegen den Anti-DNA-Bindungsprotein-Antikörper in dem in Stufe a) erhaltenen Komplex gerichtet ist.

4. Verfahren nach einem der Ansprüche 1 bis 3, wobei ein Kernextrakt einer Zellinie als Quelle für das DNA-Bindungsprotein verwendet wird.

5. Verfahren nach einem der Ansprüche 1 bis 4, wobei ein von Humanzellen abgeleitetes Autonom Replizierende Sequenz-DNA-Fragment, welches Affinität gegenüber dem menschlichen DNA-Bindungsprotein c-myc aufweist, erhalten wird, das unter folgenden Sequenzen: 
a)	GTATGATACAGATCGTGAGAATACGTAGCCTCGTCACCATTGAGCAGTAC
	GTTGTACTGGAAGAGACCATGCTCTGACACTGCACGACGTGACAGCATC

b)	AAGCTTGTTTTGGCCGTTTGTAGGGTTTGT
	TGGAATTTTTTTTCCGTCTGTGTACTTCG
	TCGAATTATTTCACGTTGCCATTACCGGTT
	CTCCATAGGGTGATGTTCATTAGCAGTTGG
	ATGATAGGTTATTCACATCTCTTATGGCGG
	TGAATAGTCACCTCTTGAACCACTTTTTCC
	TCCAGTAACTCCTCTTTCTTCGGACCTTCT
	GCAG

c)	CATTACCGGTTCTCCATAGGGTGATGTTCATTAGCAGTTGG
	ATGATAGGTTATTCACATCTCTTATGGCGGTAATAGTCACC
	TCTTGAACCACTTTT

d)	CATTACCGGTTCTCCATAGGGTGATGTTCATTAGCAGTTGG
	ATGATAGGTTATTCACATCTCTTATGGCGGTGAATAGT

e)	CATTACCGGTTCTCCATAGGGTGATG

f)	TATTCACATCTCTTATGGCGGTGAATAGT

g)	GTGAATAGTCACCTCTTGAACCACTTTT

h)	TATTCACATCTCTTATGGCGGTGAATAGTCACCTCTTGAACCACTTTT

i)	ATCGTGAGAATACGTAGCCTCGTCACCATTGAGCAGTACGTTGTACTG

k)	GAGAATACGTAGCCTCGTCACCATTGAGCAGTACGTTGTACT

	GGAAGAGACCATGCTC
 und deren funktionellen Äquivalenten ausgewählt ist.

6. Verfahren zur Herstellung eines Plasmids, wobei das Verfahren die Bereitstellung eines Plasmids umfaßt, welches ein von Säugerzellen abgeleitetes Autonom Replizierende Sequenz-DNA-Fragment gemäß Anspruch 5, einen Promoter und ein Gen zur Peptidproduktion, einschließlich des Translationsinitiationskodons, enthält.

7. Verfahren zur Herstellung eines Peptids, wobei man in Zellen ein Plasmid propagiert, das ein von Säugerzellen abgeleitetes Autonom Replizierende Sequenz-DNA-Fragment gemäß Anspruch 5, einen Promoter und ein Gen zur Peptidproduktion, einschließlich des Translationsinitiationskodons, enthält.

8. Verfahren zur Herstellung einer transfizierten Säugerzelle, wobei das Verfahren die Transfektion einer Säugerzelle mit einem Plasmid umfaßt, welches ein von Säugerzellen abgeleitetes Autonom Replizierende Sequenz-DNA-Fragment gemäß Anspruch 5, einen Promoter und ein Gen zur Peptidproduktion, einschließlich des Translationsinitiationskodons, enthält.

9. Verfahren zur Herstellung eines Plasmids, welches ein von Säugerzellen abgeleitetes Autonom Replizierende Sequenz-DNA-Fragment enthält, wobei das Verfahren folgende Schritte umfaßt:
a) Behandlung des menschlichen c-myc-Gens mit HindIII und PstI;
b) Abtrennung des stromaufwärts gelegenen HindIII-PstI-Fragmentes; und
c) Subklonierung des Fragmentes in pUC19 an der entsprechenden Stelle.

10. Verfahren zur Herstellung eines von Säugerzellen abgeleiteten Autonom Replizierende Sequenz-DNA-Fragments, wobei das Verfahren folgende Schritte umfaßt:
a) Ausschneiden des HindIII-PstI-Fragmentes mit einer Größe von etwa 200 bp aus dem nach Anspruch 9 erhältlichen Plasmid; und
b) Abtrennung des Fragmentes durch Bildung eines Komplexes, der das c-myc-Protein umfaßt.

11. Verfahren nach Anspruch 5 oder 10, wodurch ein ARS-DNA-Fragment, gekennzeichnet durch die Sequenz GTGAATAGT, erhältlich ist.

12. Verfahren zur Herstellung eines Peptids, welches die Propagierung eines Plasmids gemäß Anspruch 9 in Zellen umfaßt.

13. Verfahren zur Herstellung einer transfizierten Säugerzelle, wobei das Verfahren die Transfektion einer Säugerzelle mit einem Plasmid gemäß Anspruch 9 umfaßt.

## Revendications (Revendications pour l'(les) Etat(s) contractant(s) suivant(s): BE, CH, DE, FR, GB, GR, IT, LI, NL, SE)

1. Procédé de récupération d'un fragment d'ADN à séquence ARS provenant d'une cellule mammalienne, lequel comprend la fixation d'un fragment d'ADN provenant d'une cellule mammalienne à une protéine fixant l'ADN, la séparation du complexe ADN/protéine fixant l'ADN et l'isolement de l'ADN dudit complexe ADN/protéine fixant l'ADN,
dans lequel la protéine fixant l'ADN est sélectionnée dans le groupe constitué par les protéines myc, la protéine c-myb, la protéine v-myb, la protéine c-fos, la protéine v-fos et p 53 et dans lequel l'ADN à séquence ARS a pour origine une lignée cellulaire mammalienne produisant ladite protéine fixant l'ADN à une concentration élevée.

2. Procédé selon la revendication 1, dans lequel
a) avant l'étape de séparation le complexe ADN/protéine fixant l'ADN est en outre fixé à un anticorps anti-protéine fixant l'ADN dans le but d'obtenir un complexe ADN/protéine fixant l'ADN/anticorps anti-protéine fixant l'ADN et
b) le complexe obtenu à l'étape a) est en outre fixé à une substance capable de se fixer spécifiquement à l'anticorps du complexe.

3. Procédé selon la revendication 2, dans lequel la substance telle qu'appliquée à l'étape b) est sélectionnée dans le groupe constitué par la protéine A et un anticorps dirigé contre l'anticorps anti-protéine fixant l'ADN dans le complexe obtenu à l'étape a).

4. Procédé selon une des revendications 1 à 3, dans lequel un extrait nucléaire d'une lignée cellulaire est utilisé comme source de protéine fixant l'ADN.

5. Fragment d'ADN à séquence ARS provenant d'une cellule humaine et ayant une affinité pour la protéine humaine fixant l'ADN c-myc, sélectionné dans le groupe constitué par les séquences suivantes : 
a)
GTATGATACAGATCGTGAGAATACGTAGCCTCGTCACCATTGAGCAGTAC
GTTGTACTGGAAGAGACCATGCTCTGACACTGCACGACGTGACAGCATC
 
b)	AAGCTTGTTTGGCCGTTTGTAGGGTTTGT
	TGGAATTTTTTTTCCGTCTGTGTACTTCG
	TCGAATTATTTCACGTTGCCATTACCGGTT
	CTCCATAGGGTGATGTTCATTAGCAGTTGG
	ATGATAGGTTATTCACATCTCTTATGGCGG
	TGAATAGTCACCTCTTGAACCACTTTTTCC
	TCCAGTAACTCCTCTTTCTTCGGACCTTCT
	GCAG

c)	CATTACCGGTTCTCCATAGGGTGATGTTCATTAGCAGTTGG
	ATGATAGGTTATTCACATCTCTTATGGCGGTAATAGTCACC
	TCTTGAACCACTTTT

d)	CATTACCGGTTCTCCATAGGGTGATGTTCATTAGCAGTTGG
	ATGATAGGTTATTCACATCTCTTATGGCGGTGAATAGT

e)	CATTACCGGTTCTCCATAGGGTGATG

f)	TATTCACATCTCTTATGGCGGTGAATAGT

g)	GTGAATAGTCACCTCTTGAACCACTTTT

h)	TATTCACATCTCTTATGGCGGTGAATAGTCACCTCTTGAACCACTTTT

i)	ATCGTGAGAATACGTAGCCTCGTCACCATTGAGCAGTACGTTGTACTG

k)	GAGAATACGTAGCCTCGTCACCATTGAGCAGTACGTTGTACT

	GGAAGAGACCATGCTC
 et leurs équivalents fonctionnels.

6. Plasmide contenant un fragment d'ADN à séquence ARS provenant d'une cellule mammalienne selon la revendication 5, un promoteur et un gène de production de peptide, y compris le codon d'initiation de la traduction.

7. Procédé de production d'un peptide, lequel comprend la multiplication dans des cellules d'un plasmide contenant un fragment d'ADN à séquence ARS provenant d'une cellule mammalienne selon la revendication 5, un promoteur et un gène de production de peptide, y compris le codon d'initiation de la traduction.

8. Cellule mammalienne transfectée par un plasmide contenant un fragment d'ADN à séquence ARS provenant d'une cellule mammalienne selon la revendication 5, un promoteur et un gène de production de peptide, y compris le codon d'initiation de la traduction.

9. Plasmide contenant un fragment d'ADN à séquence ARS provenant d'une cellule mammalienne, qu'il est possible d'obtenir par
a) traitement du gène humain c-myc par HindIII et PstI ;
b) séparation du fragment amont HindIII-PstI et
c) sous-clonage du fragment dans pUC19 au site correspondant

10. Fragment d'ADN à séquence ARS provenant d'une cellule mammalienne, qu'il est possible d'obtenir par
a) excision du fragment HindIII-PstI d'environ 200 paires de base du plasmide qu'il est possible d'obtenir selon la revendication 9 et
b) séparation du fragment par formation d'un complexe comprenant la protéine c-myc.

11. Fragment d'ADN à séquence ARS selon la revendication 5 ou 10, caractérisé par la séquence GTGAATAGT.

12. Procédé de production d'un peptide, lequel comprend la multiplication dans des cellules d'un plasmide selon la revendication 9.

13. Cellule mammalienne transfectée par un plasmide selon la revendication 9.

## Revendications (Revendications pour l'(les) Etat(s) contractant(s) suivant(s): AT, ES)

1. Procédé de récupération d'un fragment d'ADN à séquence ARS provenant d'une cellule mammalienne, lequel comprend la fixation d'un fragment d'ADN provenant d'une cellule mammalienne à une protéine fixant l'ADN, la séparation du complexe ADN/protéine fixant l'ADN et l'isolement de l'ADN dudit complexe ADN/protéine fixant l'ADN,
dans lequel la protéine fixant l'ADN est sélectionnée dans le groupe constitué par les protéines myc, la protéine c-myb, la protéine v-myb, la protéine c-fos, la protéine v-fos et p 53 et dans lequel l'ADN à séquence ARS a pour origine une lignée cellulaire mammalienne produisant ladite protéine fixant l'ADN à une concentration élevée.

2. Procédé selon la revendication 1, dans lequel
a) avant l'étape de séparation le complexe ADN/protéine fixant l'ADN est en outre fixé à un anticorps anti-protéine fixant l'ADN dans le but d'obtenir un complexe ADN/protéine fixant l'ADN/anticorps anti-protéine fixant l'ADN et
b) le complexe obtenu à l'étape a) est en outre fixé à une substance capable de se fixer spécifiquement à l'anticorps du complexe.

3. Procédé selon la revendication 2, dans lequel la substance telle qu'appliquée à l'étape b) est sélectionnée dans le groupe constitué par la protéine A et un anticorps dirigé contre l'anticorps anti-protéine fixant l'ADN dans le complexe obtenu à l'étape a).

4. Procédé selon une des revendications 1 à 3, dans lequel un extrait nucléaire d'une lignée cellulaire est utilisé comme source de protéine fixant l'ADN.

5. Procédé selon une des revendications 1 à 4, dans lequel est obtenu un fragment d'ADN à séquence ARS provenant d'une cellule humaine et ayant une affinité pour la protéine humaine fixant l'ADN c-myc, sélectionné dans le groupe constitué par les séquences suivantes: 
a)
GTATGATACAGATCGTGAGAATACGTAGCCTCGTCACCATTGAGCAGTAC
GTTGTACTGGAAGAGACCATGCTCTGACACTGCACGACGTGACAGCATC
 
b) AAGCTTGTTTTGGCCGTTTGTAGGGTTTGT
TGGAATTTTTTTTCCGTCTGTGTACTTCG
TCGAATTATTTCACGTTGCCATTACCGGTT
CTCCATAGGGTGATGTTCATTAGCAGTTGG
ATGATAGGTTATTCACATCTCTTATGGCGG
TGAATAGTCACCTCTTGAACCACTTTTTCC
TCCAGTAACTCCTCTTTCTTCGGACCTTCT
GCAG

c) CATTACCGGTTCTCCATAGGGTGATGTTCATTAGCAGTTGG
ATGATAGGTTATTCACATCTCTTATGGCGGTAATAGTCACC
TCTTGAACCACTTTT

d) CATTACCGGTTCTCCATAGGGTGATGTTCATTAGCAGTTGG
ATGATAGGTTATTCACATCTCTTATGGCGGTGAATAGT

e) CATTACCGGTTCTCCATAGGGTGATG

f) TATTCACATCTCTTATGGCGGTGAATAGT

g) GTGAATAGTCACCTCTTGAACCACTTTT

h) TATTCACATCTCTTATGGCGGTGAATAGTCACCTCTTGAACCACTTTT

i) ATCGTGAGAATACGTAGCCTCGTCACCATTGAGCAGTACGTTGTACTG

k) GAGAATACGTAGCCTCGTCACCATTGAGCAGTACGTTGTACT

GGAAGAGACCATGCTC
 et leurs équivalents fonctionnels.

6. Procédé de production d'un plasmide, lequel comprend la production d'un plasmide contenant un fragment d'ADN à séquence ARS provenant d'une cellule mammalienne selon la revendication 5, un promoteur et un gène de production de peptide, y compris le codon d'initiation de la traduction.

7. Procédé de production d'un peptide, lequel comprend la multiplication dans des cellules d'un plasmide contenant un fragment d'ADN à séquence ARS provenant d'une cellule mammalienne selon la revendication 5, un promoteur et un gène de production de peptide, y compris le codon d'initiation de la traduction.

8. Procédé de production d'une cellule mammalienne transfectée, lequel comprend la transfection d'une cellule mammalienne par un plasmide contenant un fragment d'ADN à séquence ARS provenant d'une cellule mammalienne selon la revendication 5, un promoteur et un gène de production de peptide, y compris le codon d'initiation de la traduction.

9. Procédé de production d'un plasmide contenant un fragment d'ADN à séquence ARS provenant d'une cellule mammalienne, lequel comprend les étapes de
a) traitement du gène humain c-myc par HindIII et PstI;
b) séparation du fragment amont HindIII-PstI et
c) sous-clonage du fragment dans pUC19 au site correspondant.

10. Procédé de production d'un fragment d'ADN à séquence ARS provenant d'une cellule mammalienne, lequel comprend les étapes
a) d'excision du fragment HindIII-PstI d'environ 200 paires de base du plasmide qu'il est possible d'obtenir selon la revendication 9 et
b) de séparation du fragment par formation d'un complexe comprenant la protéine c-myc.

11. Procédé selon la revendication 5 ou 10, par lequel un fragment d'ADN à séquence ARS, caractérisé par la séquence GTGAATAGT, est obtenu.

12. Procédé de production d'un peptide, lequel comprend la multiplication des cellules d'un plasmide selon la revendication 9.

13. Procédé de production d'une cellule mammalienne transfectée, lequel comprend la transfection d'une cellule mammalienne par un plasmide selon la revendication 9.
